(19) Europäisches Patentamt
European Patent Office
Office européen des brevets

(11) **EP 4 085 891 A1**

(12) **EUROPEAN PATENT APPLICATION**

(43) Date of publication:
**09.11.2022 Bulletin 2022/45**

(51) International Patent Classification (IPC):
**A61J 1/00** (2006.01)    **A61K 31/4174** (2006.01)

(21) Application number: **21172306.9**

(52) Cooperative Patent Classification (CPC):
**A61J 1/00; A61K 9/0019; A61K 9/08; A61K 31/4174**

(22) Date of filing: **05.05.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **B. Braun Melsungen AG**
**34212 Melsungen (DE)**

(72) Inventors:
• **ADAMO, Vincent**
**1723 Marly (CH)**

• **JUIF, Olivier**
**01630 Peron (FR)**
• **CLAUDET, Marie-Celine**
**25240 Sarrageois (FR)**
• **TIMONEDA RAMIA, Cristina**
**08392 Sant Andreu de Llavaneres (ES)**

(74) Representative: **Prüfer & Partner mbB**
**Patentanwälte · Rechtsanwälte**
**Sohnckestraße 12**
**81479 München (DE)**

(54) **DEXMEDETOMIDINE-SOLUTION IN A LOW-DENSITY POLYETHYLENE CONTAINER**

(57)    The present invention refers to a container comprising a compartment filled with an aqueous solution of dexmedetomidine and optionally closed with a cap, wherein at least the inner surface of the compartment which is in contact with the aqueous solution of dexmedetomidine, is made of low density polyethylene (LDPE). The present invention further refers to the use of said container for storing the aqueous dexmedetomidine solution. Finally, the present invention refers to a method for manufacturing a container filled with an aqueous solution of dexmedetomidine comprising the steps of providing the container, filling the compartment of the container with an aqueous dexmedetomidine solution, sealing the compartment of the container, and optionally providing the container with a cap.

**Fig. 1**

EP 4 085 891 A1

**Description**

Field of the invention

The present invention refers to a container comprising a compartment filled with an aqueous solution of dexmedetomidine and optionally closed with a cap, wherein at least the inner surface of the compartment which is in contact with the aqueous solution of dexmedetomidine, is made of low density polyethylene (LDPE). The present invention further refers to the use of said container for storing the aqueous dexmedetomidine solution. Finally, the present invention refers to a method for manufacturing a container filled with an aqueous solution of dexmedetomidine comprising the steps of providing the container, filling the compartment of the container with an aqueous dexmedetomidine solution, sealing the compartment of the container, and optionally providing the container with a cap.

Background of the invention

Dexmedetomidine is a known pharmaceutically active compound. It has medical effects, which includes reducing anxiety and acting as sedative and/ or as analgesic respectively for pain medication. Dexmedetomidine is commercially available e.g. as a solution for injection in various containers such as a glass vial, to be used after dilution, or ready-to-use.

Diluting the dexmedetomidine-solution prior to its use, as well as the presence of the dexmedetomidine-solution in glass containers is associated with multiple disadvantages or problems, e.g. with the risk of handling errors (such as calculation errors, or glass splinters), and/or with the risk of contamination, such as microbiological contamination, for instance due to micro-cracks in the glass container or from dilution steps.

In this regard, US 10,632,043 discloses a single layer or multilayered film pack comprising a polypropylene homopolymer contact layer for packaging premixed (ready-to-use) injectable dexmedetomidine formulations. Also mentioned as comparative example is a multi-layered bag having HDPE/LLDPE/HDPE layer (Table 4). The disclosed film-bags are not self-supporting.

WO 2019/164765 also discloses ready-to-use injectable compositions comprising dexmedetomidine, where the finished dosage form is provided in a sealed polymeric container, and wherein this polymeric container comprises a cyclic olefin polymer. Further containers are described in US 9,320,712 B2, US 9,649,296 B1, US 9,717,796 B1, WO 2017/184188 A1, JP5921928 B2, and US 2020138788 A1.

Moreover, depending on the respective material of the container (and, as the case may be, of the cap and/or of a sealing underneath the cap), there is the risk of leachable compounds which potentially (further) contribute to contamination of the aqueous dexmedetomidine-solution. Additionally, in particular when aiming at providing ready-to-use solutions, it may be the case that the respective pharmaceutically active ingredient, i.e. here the API dexmedetomidine, is present in these solutions in a comparably low concentration (especially when compared to the respective non-diluted composition). This can result in a sorption of the API (active pharmaceutical ingredient) dexmedetomidine to the inner wall of the compartment of the container comprising the API, thereby, in turn, amongst others resulting in administration of incorrect amounts of API to a patient. The problem of sorption is particularly known for dexmedetomidine solutions, which are known to have a strong tendency to be either adsorbed onto or absorbed into plastic materials.

Thus, there exists a need of providing improved containers comprising aqueous solutions containing dexmedetomidine, in particular for ready-to-use (RTU) applications (also referred to as "premix"), with improvements for example with regard to handling and/or avoiding or lowering impurities, and optionally at the same time reducing the sorption of the dexmedetomidine to the inner wall of the container.

Summary of the invention

It has unexpectedly been found that a container comprising a compartment that is filled with an aqueous solution of dexmedetomidine, wherein at least the entire surface of the compartment which may come into contact with the aqueous solution of dexmedetomidine at least during upright position, is made of low density polyethylene (LDPE), overcomes one or more of the disadvantages and problems referred to herein when used for storing the aqueous solution of dexmedetomidine. In particular, it has been found that the combination of the specific material of the container and the ready-to-use ("RTU")-aqueous solution that contains the API (active pharmaceutical ingredient) dexmedetomidine in a comparably low concentration, can substantially contribute to reduce or even avoid contaminations (such as microbial contaminations or contaminations due to glass splinters). Since a RTU application is safe, a further dilution is not necessary prior to administering the aqueous dexmedetomidine-solution, and thereby the risk of handling errors can additionally be reduced.

Thus, in one aspect, the present invention refers to a container comprising a compartment filled with an aqueous solution of dexmedetomidine, wherein at least the inner surface of the compartment which may come in contact with the aqueous solution of dexmedetomidine is made of low density polyethylene (LDPE).

It was surprisingly found that if the compartment of the container that is filled with an aqueous solution of dexmedetomidine is made from LDPE, i.e. at least the inner surface of the compartment which is in contact with the dexmedetomidine solution, and preferably the entire compartment or entire container (including or excluding stoppers and caps) is made of LDPE, this results in a low level of leachables and low sorption (absorption and/or adsorption) of the API dexmedetomidine into and/or onto the compartment's inner surface. This is all the more surprising as dexmedetomidine is known to have a strong tendency to be adsorbed onto or absorbed into plastic materials.

It has additionally been surprisingly found that an aqueous solution of dexmedetomidine also has a high long term stability in the container of the present invention, for example at least 2 years at room temperature. Compared to known glass bottles, the container can hardly break, and if it breaks, there are no glass splinters present. This provides for an advantageous handling, as the occurrence of splinters such as glass splinters (as it is the case if glass bottles break) is avoided.

In a further aspect, the present invention refers to the use of the container according to the present invention for storing an aqueous dexmedetomidine solution.

In another aspect, the present invention refers to a method for manufacturing a container comprising a sealed compartment filled with an aqueous solution of dexmedetomidine, comprising a step of providing a container of the invention, filling the compartment of the container with the aqueous dexmedetomidine-solution, and sealing the compartment of the container with or without a sealing member, and optionally providing the container with a stopper and/or cap.

In a further aspect of the present invention, the premixed aqueous solution of dexmedetomidine, filled in a compartment of a container such that the surface of the compartment wall which may come into contact with the aqueous solution of dexmedetomidine is made of low density polyethylene (LDPE), is used in a medical treatment, prophylactically and/or therapeutically. The useful medical treatment is typically selected from reducing anxiety, acting as sedative and acting as analgesic.

Brief description of the drawings

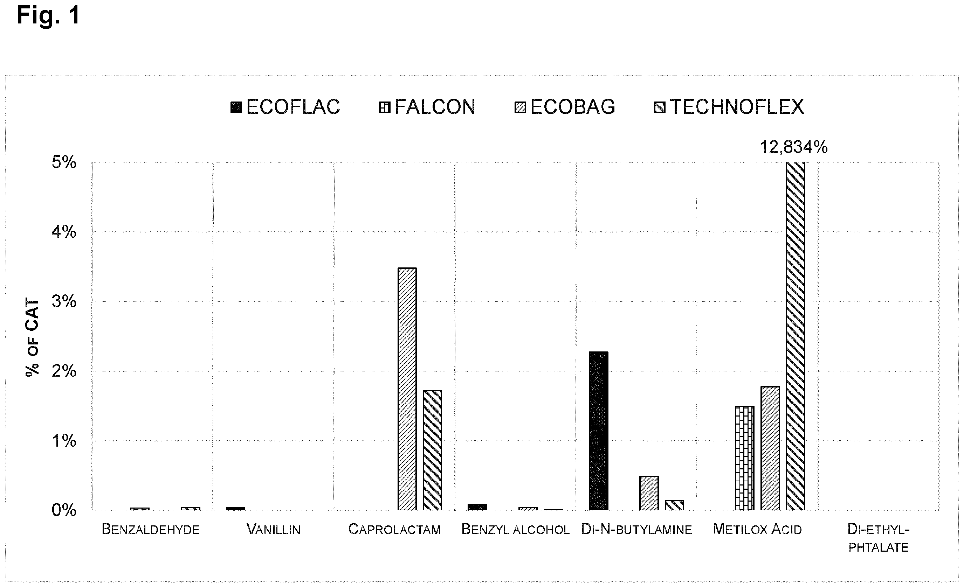

Fig.1: Figure 1 shows the concentration levels as % of the "Compound Analytical Threshold" CAT for each already identified leachables in different container closure systems, which includes a comparison with a container according to an embodiment of the present invention, after a storage of 12 months at 25°C. The parameter "% of CAT" takes into consideration both concentration and toxicological level aspects. In figure 1 the following values are depicted:

| Leachable compounds | T12 months stored at 25°C/60%RH | | | | |
|---|---|---|---|---|---|
| | ECOFLAC | ECOFLAC | BFlex | ECOBAG | TechnoFlex |
| Benzaldehyde | 0.01 | 0.00 | 0.04 | 0.00 | 0.04 |
| Vanillin | 0.60 | 0.04 | 0.00 | 0.00 | 0.00 |
| Caprolactam | 0.00 | 0.00 | 0.00 | 3.48 | 1.72 |
| Benzyl alcohol | 0.45 | 0.09 | 0.00 | 0.04 | 0.01 |
| Di-N-butylamine | 3.93 | 2.27 | 0.00 | 0.48 | 0.14 |
| Metilox Acid | N.A. | N.A. | 1.49 | 1.77 | 12.83 |
| Di-ethyl-phtalate | 0.004 | 0.00 | 0.00 | 0.00 | 0.00 |

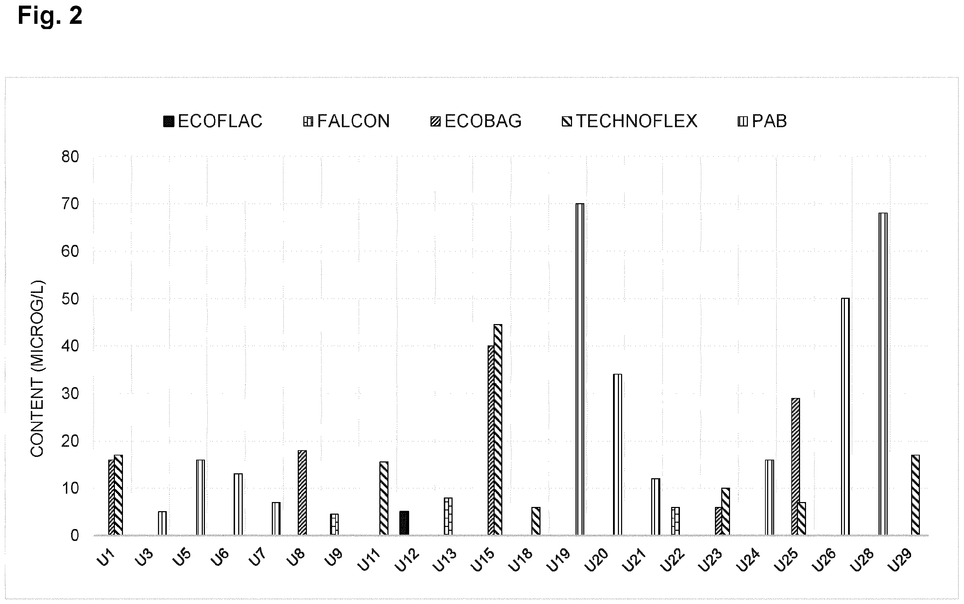

Fig. 2: Figure 2 shows the concentration levels expressed in $\mu$g/l of unknown leachables in different container closure systems, which includes a comparison with a container according to an embodiment of the present invention, after a storage of 12 months at 25°C.

Detailed description of the invention

The present invention relates to the following embodiments:

1. Container, preferably bottle with bottle head/neck, preferably having a neck/tapered end portion, comprising a compartment filled with an aqueous solution of dexmedetomidine, wherein at least the inner surface of said compartment which is in contact with the aqueous solution of dexmedetomidine, when the container is in upright position, is made of low density polyethylene (LDPE).

2. Container according to item 1, which is self-supporting and/or whose compartment is exclusively made of a single layer of low density polyethylene (LDPE).

3. Container according to item 1 or 2, wherein said LDPE has a density in the range of from 0.910-0.937 g/cm$^3$.

4. Container according to any of the preceding items, which is provided with a cap, wherein preferably the cap comprises an elastomer, further preferred, the cap consists of a high density polyethylene (HDPE) shell, a thermoplastic elastomer stopper and an HDPE attachment (prepared by ring molding).

5. Container according to any of the preceding items, wherein the entire surface of the compartment of the container which can come into contact with aqueous solution of dexmedetomidine in any position of the container is made of LDPE.

6. Container according to any of the preceding items, wherein the compartment of the container is sealingly closed.

7. Container according to any of the preceding items, wherein

(i) the entire inner layer of the sealed compartment of the container is made of LDPE, or
(ii) the sealed compartment of the container consists of a single LDPE layer, which means that the compartment has a single wall consisting of a single LDPE layer, or
(iii) a separate sealing member (such as a membrane or sealing foil) is attached for closure of compartment of the container, wherein preferably the separate sealing member has an inner layer made of LDPE, or the sealing member consists of LDPE.

8. Container according to item 6 or 7, wherein the compartment of the container is sealingly closed by a membrane, wherein the membrane is preferably formed as an integral part of the container using a BFS (blow-fill-seal) process.

9. Container according to item 8, wherein the membrane is a separately formed sealing member for sealing the container neck after filling.

10. Container according to item 8 or 9, wherein the membrane is part of the cap and separates the packed fluid from elastomeric components during storage/shelf-life.

11. Container according to any of the preceding items, wherein the LDPE is free of stabilizers and plasticizers, in particular the LDPE is free of any additives.

12. Container according to any of the preceding items, which is made by applying an injection-molding technique, or a blow-molding technique, preferably the container is made by applying the blow-fill-seal technique.

13. Container according to any of the preceding items, having a compartment with a volume in the range of between 10 ml and 500 ml.

14. Container according to any of the preceding items, wherein the compartment of the container has a wall thickness of at least 200 μm, preferably of at least 250 μm.

15. Container according to any of the preceding items, wherein the aqueous dexmedetomidine-solution has a contact surface with an inner side of the compartment of the container in the range of 0.6 cm$^2$/ml to 1.8 cm$^2$/ml, preferably in the range of from 1.2 cm$^2$/ml to 1.4 cm$^2$/ml.

16. Container according to any of the preceding items, wherein the aqueous dexmedetomidine solution is an aqueous dexmedetomidine/NaCl-solution, preferably the NaCl-solution is 0.5% to 1.0% weight/volume.

17. Container according to any of the preceding items, wherein the aqueous dexmedetomidine solution preferably is a premixed, ready-to-use dexmedetomidine solution, more preferably a ready-to-use injectable or infusable solution.

18. Container according to any of the preceding items, wherein the aqueous dexmedetomidine-solution has a concentration of dexmedetomidine from 2 μg/ml to 12 μg/ml, preferably the concentration of dexmedetomidine is 4 μg/ml to10 μg/ml, more preferably the concentration of dexmedetomidine is 4 μg/ml or 8 μg/ml.

19. Container according to any of the preceding items, wherein the concentration of dexmedetomidine is 4 μg/ml or 8 μg/ml, preferably the concentration of dexmedetomidine is 4 μg/ml.

20. Use of a container comprising a compartment with an inner wall surface made of low density polyethylene (LDPE), or comprising a compartment made of low density polyethylene (LDPE), respectively for storing an aqueous dexmedetomidine-solution, preferably an aqueous dexmedetomidine-solution having a concentration of dexmedetomidine in the range of from 4 μg/ml to 8 μg/ml, preferably the concentration of dexmedetomidine is 4 μg/ml or 8 μg/ml, more preferably the concentration of dexmedetomidine is 4 μg/ml.

21. Method for manufacturing a container comprising a sealed compartment filled with an aqueous solution of dexmedetomidine, wherein the container is optionally provided with a cap, comprising the following steps:

(i) Providing a container comprising a compartment comprising an inner surface made of low density polyethylene (LDPE), preferably by applying a blow-molding process or an injection-molding process;
(ii) Filling the compartment of the container with the aqueous solution of dexmedetomidine, preferably with the aqueous solution of dexmedetomidine as disclosed herein;

(iii) Sealing the filled compartment of the container.

22. Method according to item 21, wherein a blow-fill-seal technique is applied for steps (i) to (iii).

23. Method according to item 21 or 22, further comprising an additional step (iv) of sterilizing the filled, sealed and optionally locked container, followed by a step (v) of labelling the container, thereby obtaining the final, ready-to-use container (finished medicinal product).

24. Method according to anyone of items 21 to 23, wherein the container is as defined in any of items 1 to 15, and/or the aqueous solution of dexmedetomidine is as defined in any of items 16 to 19.

25. Premixed aqueous solution of dexmedetomidine, filled in a container comprising a compartment such that the inner surface of the compartment of the container which may come into contact with the aqueous solution of dexmedetomidine is made of low density polyethylene (LDPE), for use in a medical treatment.

26. Premixed aqueous solution of dexmedetomidine for use according to item 25, wherein the medical treatment is selected from reducing anxiety, acting as sedative and acting as analgesic, in particular for sedation of non-intubated adult patients prior to and/or during diagnostic or surgical procedures requiring sedation, and/or for sedation of adult Intensive Care Unit patients requiring a sedation level not deeper than arousal in response to verbal stimulation.

The compartment of the container of the invention comprises an inner surface which comes into contact with the filled aqueous solution of dexmedetomidine (contact surface), and which inner surface is made of low density polyethylene ("LDPE"). The compartment of the container may be sealed by the same wall material (i.e. the whole sealed compartment is formed by an inner layer or a single layer wall of LDPE); or it may be closed with a separate sealing member and/or a cap made with inner surface of LDPE too. Optionally the container may be sealed with a sealing foil or membrane as the separate sealing member, optionally underneath the optional cap. If a sealing foil or membrane is present, also this sealing foil/membrane, at least the inner surface thereof forming the compartment, can be made of LDPE. In other words, at least the entire surface of the compartment of the container, and of the sealing member if present, that is or can potentially come into contact with the aqueous solution of dexmedetomidine (e.g. if the container filled with the aqueous dexmedetomidine-solution is stored upside-down, or is shaken, or is on the side) is made of LDPE.

The term "dexmedetomidine" as used herein refers to ((+)-4-(S)-[1-(2,3-dimethylphenyl)ethyl]-1H-imidazole monohydrochloride/ 5-[(1S)-1-(2,3-dimethylphenyl)ethyl]-1*H-imidazole*) and comprises all pharmaceutically acceptable salts, preferably dexmedetomidine HCl, prodrugs, complexes, the free base form, as well as any combinations thereof. The aqueous solution of dexmedetomidine as used herein preferably comprises less than 1% of levomedetomidine, based on the weight of the dexmedetomidine. Levomedetomidine is 4-[(1R)-1-(2,3-dimethylphenyl)ethyl]-1H-imidazole. The enantiomeric purity of dexmedetomidine is preferably at least 99%, as preferably determined by HPLC (high performance liquid chromatography).

Since at least the contact surface of the compartment of the container, preferably the entire inner surface of the compartment of the container, as well as the optional sealing member (at least the inner surface thereof forming the compartment), if present, are made of LDPE, the aqueous solution of dexmedetomidine comes into contact only with LDPE at least when the container is in upright position. In one embodiment, the compartment of the container which is filled with aqueous solution of dexmedetomidine as described herein, is sealed with a separate sealing member, such as a membrane or a sealing foil. The sealing foil or membrane can be covered by a cap. In other words, the liquid in the inside of the compartment of the container (the aqueous solution of dexmedetomidine) is enclosed by the inner wall/layer/surface of the compartment of the container, and the inner side of the sealing member, if present.

In a further/preferred embodiment, the compartment of the container (and, if present, the sealing member) is preferably - besides the inner wall surface of the compartment - made from LDPE only, i.e. the compartment does not contain any amounts of polypropylene (PP), very low density polyethylene (VLDPE), linear low density polyethylene (LLDPE), high density polyethylene (HDPE) or other polymers. In this embodiment, the compartment wall is made of a single layer only, distinct from multilayer walls such as an inner layer of LDPE and an outer layer made from PP. In other words, such single-constituting compartment wall layer contains LDPE only (it consists of LDPE). The container/compartment does not have multiple layers which each layer consisting of a different polymer. In a preferred embodiment, the sealing foil also consists of only one layer, and this layer is made from LDPE only.

Accordingly, the LDPE constitutes the compartment (packaging) for the aqueous solution of dexmedetomidine, and the aqueous dexmedetomidine-solution is in contact with the LDPE at least when the container stands upright/vertical (opening/closure/neck of the container is opposite/above the aqueous solution of dexmedetomidine) or is turned downwards (contact with a sealing foil). In any case, the aqueous solution can only come into contact with LDPE.

The expression "at least the inner surface of the compartment which is in contact with the aqueous solution of dexmedetomidine" refers to an (upright) position of the container where the opening/closure/neck of the container (irrespective of whether it is closed with a cap) is opposite to the aqueous solution. This means that the expression "at least the inner surface which is in contact with the aqueous solution of dexmedetomidine" encompasses embodiments where the entire inner surface of the closed/sealed compartment is made of LDPE or the inner surface without the surface which closes the opening of the compartment. The surface of the compartment which is in contact with the aqueous solution in position can also be referred to as "contact surface".

In a preferred embodiment, the density of the LDPE is 0.910-0.937 g/cm$^3$. The test method for density is based on the Archimedes principle described in ISO 1183-1 method A. Further characteristics of the LDPE are described in Europ. Pharmacopoeia 10$^{th}$ edition, chapter 3.1.4.

In a preferred embodiment, the container is self-supporting, preferably the container is a bottle, in particular a bottle for infusion. It is also possible that the container comprises a thread to which the cap can be screwed. If a sealing foil or sealing membrane is present, this sealing foil/membrane is placed underneath the cap.

The container can be prepared by using LDPE-type polyethylene without additives, wherein the polyethylene is polymerized under high pressure in the presence of oxygen or free-radical forming initiators as catalyst.

The compartment of the container can be sealed with or without a sealing member. In a blow-fill-seal process, the compartment and the closure of the compartment (preferably with a neck) is made of the same material, i.e. without using a separate sealing member. In the blow-fill-seal process, the container/ compartment is blown, directly filled and then sealed (by welding) without further components in one process (e.g. two walls of the compartment are molten and welded together). Such an embodiment is used for EcoflacPlus® or Miniplasco®.

In another embodiment, the compartment is sealed with an additional component (made of (LD)PE), such as a foil or a moulded part whose inner surface which can come into contact with the solution is also made of (LD)PE.

In another embodiment, the compartment is sealed with a port system assembly wherein at least one component, which does not form the inner surface of the compartment, is not made of PE (e.g. stopper made of elastomeric material).

In one embodiment, the container is produced by blow-molding technology or injection-molding technology. It is additionally preferred that the corresponding container is a closed system, preferably sealed with a sealing foil or sealing membrane, so that the (preferably sealed) container is exclusively constituted of LDPE.

For bags, in prior art usually a plastic film is welded to a connector system including generally a rubber stopper which is generally directly in contact with the aqueous product solution. This direct contact leads to higher levels of leachables (leachable compounds) and potentially increased drug ad- and absorption. However, contrary thereto, for the container of the present invention, there is no contact of the solution with any other components except with LDPE at least when the container is in upright position.

Another advantage is that labelling (paper with ink) of the container of the present invention can be performed only after sterilization (e.g. autoclaving), avoiding therefore leaching components (from Ink or label paper) during this heat treatment. In case of a prior art bag, there is a printing on the primary plastic foil before the terminal sterilisation, thereby posing the risk of generating leachables during the thermal sterilisation process.

In a preferred embodiment, the LDPE is free of stabilizers and plasticizers. It is further preferred that the LDPE is free of any additives. The absence of additives can be determined by applying the test for additives based on thin-layer chromatography according to the Europ. Pharmacopoeia 10$^{th}$ edition, chapter 3.1.4, wherein additives are absent if there is only one spot for oligomers in the chromatogram.

One advantage of LDPE over HDPE is that LDPE can be prepared without any additives. The use of either LDPE with additives or HDPE with additives results in an undesirably high level of impurities in the aqueous solution of dexmedetomidine, which is present in the container. The use of LLDPE as such is disadvantageous because its melting point is too low. Consequently, certain treatments e.g., heat treatment such as sterilization, is highly disadvantageous.

It was surprising to find that a container or at least its compartment for storing the API solution can be made of polyethylene (PE), in particular LDPE, only. Usually, pharmaceutical containers such as bags are made of polypropylene (PP) or including some PE grade in the container-forming layer. The challenges of a PE container is that sterilization at 121°C (or above) causes melting/deformations, resulting in the container finally becoming opaque and hard. The PE grades that are able to overcome these challenges are also more expensive. This seems to be reason why almost all bags for intravenous infusion (i.v.) are made from PP.

Contrary thereto, the container of the present invention can be subjected to terminal sterilisation, thereby sterilizing the aqueous dexmedetomidine solution that is contained in the container. Heat sterilization is normally carried out by using steam, preferably saturated steam to allow for the use of pressure as a means of temperature control. The time period for the sterilization is chosen such that it is long enough to meet the sterility requirement of a medicinal product, e.g. an injectable or or infusable product, such as the aqueous dexmedetomidine solution that is contained in the container of the present invention.

As the container of the present invention can preferably be made self-supporting when made of LDPE, the self-supporting container can stand in filled state or, useful during the manufacturing process of the filled container, in non-filled state.

The container can comprise a thread to which the cap can be screwed. The cap can be configured to have re-sealing ability after e.g. removal of an injection needle.

In a preferred embodiment, the compartment of the container has a volume in the range of between 10 ml and 500 ml. The compartment of the container can have a wall thickness of at least 200 $\mu$m, preferably of at least 250 $\mu$m.

A preferred upper limit for the wall thickness is 2 mm, or 1 mm, preferably 500 $\mu$m.

The aqueous dexmedetomidine-solution can have a contact surface with an inner side of the compartment of the container and the sealing member, e.g., sealing foil, if present, in the range of 0.6 cm$^2$/ml to 1.8 cm$^2$/ml, preferably in the range of from 1.2 cm$^2$/ml to 1.4 cm$^2$/ml.

The compartment of the container of the present invention is filled with an aqueous solution of dexmedetomidine. The aqueous dexmedetomidine-solution is preferably a dexmedetomidine/NaCl-solution, more preferably in 0.9 % w/v NaCl. The aqueous dexmedetomidine-solution preferably has a concentration of dexmedetomidine in the range of from 4 $\mu$g/ml to 8 $\mu$g/ml, more preferably the concentration of dexmedetomidine is 4 $\mu$g/ml or 8 $\mu$g/ml. In a particular preferred embodiment, the concentration of dexmedetomidine is 4 $\mu$g/ml.

In a preferred embodiment, the aqueous dexmedetomidine-solution is a ready-to-use solution. Within the meaning of the present invention, the term "ready-to-use" ("RTU") refers to a pharmaceutical composition that is stable, is not further diluted prior to use, and is not reconstituted from a lyophilizate. The RTU pharmaceutical composition is an injectable or infusable composition that is premixed and suitable for administration to a patient without dilution. RTU pharmaceutical compositions are e.g. suitable for parenteral administration, including intravenous, subcutaneous, intramuscular and intraperitoneal administration. In a preferred embodiment, the RTU pharmaceutical composition of the present invention is suitable for intravenous administration. Hence, the aqueous dexmedetomidine solution of the present invention is preferably a ready-to-use injectable or infusable solution. It is particular preferred that the aqueous dexmedetomidine solution of the present invention is ready to use upon removing the compositions from a sealed compartment of the container.

The term "ready-to-use container" can also denote the final container of the present invention. In this context, "ready-to-use container" means that the container is filled, sealed, optionally locked, sterilized, and labelled. A "ready-to-use solution" is a composition which does not need an additional dilution step but can be directly infused.

According to the present invention, amongst other it has been surprisingly found that the aqueous dexmedetomidine solution of the present invention that is present in the container of the present invention exhibits sufficient or, in comparison with other conventional containers or bags, improved stability such as long term stability. The "stability" preferable refers to both, chemical and physical stability, of the aqueous dexmedetomidine solution. Within the meaning of the present invention, the term "stability" also means prevention of contamination by leachables (leachable products, compounds or components). Contamination by leachables can occur when containers that contain dexmedetomidine produce said leachables. Leachable compounds can be known or unknown. Examples of known leachables are benzaldehyde, vanillin, caprolactam, benzyl alcohol, di-n-butylamine, and metilox acid. It has been surprisingly found that the aqueous dexmedetomidine solution that is filled and stored in the container of the present invention exhibits sufficient stability, i.e. the concentrations of the identified leachable compounds are below the CAT ("Compound Analytical Threshold"). Hence, the aqueous solution of dexmedetomidine exhibits sufficient stability upon storage in the container of the present invention.

The invention also refers to the use of a container as described herein for storing an aqueous dexmedetomidine-solution as described herein.

Finally, the invention also refers to a method for manufacturing a container comprising a sealed compartment filled with an aqueous solution of dexmedetomidine, optionally provided with a cap, comprising the following steps:

(i) Providing a container comprising a compartment comprising an inner surface made of low density polyethylene (LDPE), preferably by applying a blow-molding process or an injection-molding process;
(ii) Filling the compartment of the container with the aqueous solution of dexmedetomidine;
(iii) Sealing the filled compartment of the container.

Optionally, the filled and sealed compartment of the container is locked e.g. with a cap.

As disclosed elsewhere herein, the container of the present invention can be subjected to heat treatment such as sterilization, as LDPE can withstand the heat that is applied during terminal sterilization procedure in an autoclave. Thus, in a preferred embodiment, the method for manufacturing the container of the present invention comprises an additional step (iv) of sterilizing the filled, sealed and optionally locked container, optionally followed by a further step (v) of labelling said container, thereby forming the final, ready-to-use (finished medicinal product). Labelling the container only after sterilization (e.g. autoclaving) offers the advantage that no leaching components (e.g. from paper and/or ink) during heat treatment contaminate the container and/or the aqueous dexmedetomidine solution.

The container at least in part made of LDPE, and preferably produced by applying a blow-molding process, is in a next step filled with the aqueous solution of dexmedetomidine. In a preferred embodiment, the compartment of the container filled with the aqueous dexmedetomidine solution is prepared by applying blow-fill-seal technology. The basic concept of blow-fill-seal is that a container comprising a compartment is formed, filled, and sealed in a continuous process without human intervention. Advantageously, the container of the present invention, containing the aqueous dexmedetomidine solution of the present invention, can be prepared in an aseptic, sterile way. Hence, in a preferred embodiment, steps (i) to (iii) represent a blow-fill-seal method.

The container to be prepared in the method of the invention is preferably the container having the features described herein, and the aqueous solution of dexmedetomidine is preferably the aqueous solution having the features described herein.

In a further aspect of the present invention, the premixed aqueous solution of dexmedetomidine, filled in a container of the invention, is used in a medical treatment. The medical treatment is typically selected from reducing anxiety, acting as sedative and acting as analgesic. Particular advantages are exerted when the medical treatment is for sedation of non-intubated adult patients prior to and/or during diagnostic or surgical procedures requiring sedation (i.e. procedural/awake sedation), and/or for sedation of adult ICU (Intensive Care Unit) patients requiring a sedation level not deeper than arousal in response to verbal stimulation (corresponding to Richmond Agitation-Sedation Scale (RASS) of 0 to -3).

Examples

Example 1: Preparation of container

A container (Ecoflac Plus) having a monolayer wall made of only LDPE for use according to an embodiment of the present invention was prepared using a polymer material of LDPE without additives, notably free of DEHP, latex and PVC, by the blow-fill-seal technique, i.e. by a machine operation that, without interruption between steps, included blowing the container form, filling the container with the aqueous solution of dexmedetomidine. The container was closed by a cap consisting of a HDPE shell, a thermoplastic elastomer stopper and an HDPE attachment.

Example 2: Comparison of container according to the present invention and comparison containers

The following examples present a summary of representative experimental data to be used in the context of the present invention for the formulation of dexmedetomidine (DMT) at low concentration (4 $\mu$g/ml) in various plastic containers of bags. The containers tested included those listed in Table 1 below, respectively having the material constitution also indicated in Table 1.

The following Tables 1 to 6 describes the investigated plastic containers with configurations A to H for the dexmedetomidine (DMT) ready-to-use (RTU) solution, wherein configuration A is in accordance with an embodiment of the present invention (Example 1), and configurations B to H are comparisons:

Table 1: Plastic containers description evaluated for the DMT RTU formulation

| | CONTAINER | STATUS | THE MATERIAL OF THE SURFACE CONTACT | THICKNESS ($\mu$M) | CONTACT WITH STOPPER |
|---|---|---|---|---|---|
| A | container of Example 1 (Ecoflac Plus) | commercial | LDPE without additives monolayer | 250 | No |
| B | Falcon | prototype | PP/20% SEBS | 200 | No |
| C | PAB | commercial | PP/10% SEBS monolayer | 300 | Yes |
| D | Ecobag® | commercial | Polyester/PP&PE | 200 | Yes |
| E | Technoflex® | commercial | Inerta® 103, PP-based | 200 | No |
| F | Urotainer PVC free | commercial | Polyester/PP&PE | 200 | Yes |
| G | Lipex/Clinex | commercial | Ecdel/PP/40%SEBS | 252 | No |
| H | Urotainer PVC | commercial | PVC monolayer | 200 | Yes |

Table 2: Tested Ecoflac Plus container closure system (A) according to the invention - Lot 1820A-07 (62 mL filling volume)

| Packaging | Bottle | Cap | | | Label material | Label Ink | Direct contact with stopper |
|---|---|---|---|---|---|---|---|
| | | Shell | Stopper | Attachment (Ring) | | | |
| | LDPE without additives | HDPE with additives | TPE | | Self adhesive paper | UV flexo | No |
| Sterilization | | | | | | | |
| Storage | | | | | | | |

Table 3: Tested container closure system with Container (B) - Lot 1828A-01 (55 mL filling volume)

| Packaging | Primary Film | Port | Printing | Overwrap | Direct contact with stopper |
|---|---|---|---|---|---|
| | BFlex batch 1728 | Falcon port with stopper | UVflexo XYZ & Thermotransfer | BFlex SF150 | No |
| Sterilization | 121°C, F0 ≥ 15 min | | | | |
| Storage | Corrugated cardboard | | | | |

Table 4: Tested overwrapped PAB 150 mL container closure system (C) - Lot 1718A-01/3 (50 mL filling volume)

| Packaging | Primary Film | Port | Printing | Overwrap | Direct contact with stopper |
|---|---|---|---|---|---|
| | PAB Lot J6A651 | PAB commercial port system | Yes | SiOx | Yes |
| Sterilization | 121°C, F0 ≥ 15 min | | | | |
| Storage | Corrugated cardboard | | | | |

Table 5: Tested overwrapped 100 mL EcoBag container closure system (D) - Lot 1916A-EB01 (55 mL filling volume)

| Packaging | Primary Film | Port | Printing | Overwrap | Direct contact with stopper |
|---|---|---|---|---|---|
| | Mulitlayer M312 | Ecobag commercial Port system | Yes | PA6/PP | Yes |
| Sterilization | 121°C, F0 ≥ 15 min | | | | |
| Storage | Corrugated cardboard | | | | |

Table 6: Tested overwrapped 100 mL TechnoFlex i.v. bag (E), Art. 10-005 - Lot 1916A-TF01 (55 mL filling volume)

| Packaging | Primary Film | Closure system | Stopper | Printing | Overwrap (during stability) | Direct contact with stopper |
|---|---|---|---|---|---|---|
| | Inerta® 103, PP-based | Twist-off port 823 Injection site | Polyisoprene | Yes | Overwrap clear for I.V. bag | No |
| Sterilization | 121°C, F0 ≥ 15 min<br>Note: Sterilization in SiOx overwrapping pounch | | | | | |
| Storage | Corrugated cardboard | | | | | |

Analytical Method description

The assay and impurities of Dexmedetomidine (related compounds and leachables) in saline formulations (0.9% w/v NaCl) are determined by a reversed-phase UPLC with UV and ESI-MS detection. The target concentration of Dexmedetomidine free base in formulation is 4 μg/mL.

All the weighing activities of Dexmedetomidine drug substance were performed under a safety weighing enclosure system from Safetech with a calibrated analytical micro balance XPE056 from Mettler Toledo.

For the analysis, 25 μL of as-is formulation was injected via a UHPLC Gradient System (UPLC H-Class Waters with FTN injection module, 50 μL extension loop, with a PDA 25mm high sensitivity cell and QDa detector) and passed through a BEH C18 column purchased from Waters (Acquity BEH C18, 50x2.1 mm, 1.7μm, cat. n°#186002350).

Example 3: Comparison of sorption of DMT after sterilization

Table 7 shows results of DMT loss after sterilization in the 8 different plastic containers. Lowest sorption of DMT at 4 μg/mL was obtained in Ecoflac Plus 100mL container (A). A comparison over 24-month storage for five containers is presented in Table 8, and confirms good stability of DMT assay in Ecoflac Plus container.

Table 7: DMT loss after sterilization in 8 different plastic containers

| | Packaging | Estimated contact surface with film ($cm^2$/mL) | Direct contact with stopper | % Sorption after terminal sterilization |
|---|---|---|---|---|
| A | Ecoflac Plus | 1.2 | No | 0.3 |
| B | Falcon | 3.9 | No | 1.3 |
| C | PAB | 5.1 | Yes | 0.7 |
| D | Ecobag | 3.6 | Yes | 2.5 |
| E | TechnoFlex | 2.9 | No | 1.5 |
| F | UT PVC free | 3.7 | Yes | 4.1 |
| G | Lipex/Clinex | 1.1 | No | 4.6 |
| H | UT PVC | 1.6 | Yes | 16.2 |

Table 8: Comparison of assay of DMT at 4 μg/mL over 24-month for several packaging systems and Ecoflac Plus

| Time Point | Stability condition | Recovery of DMT Assay (%) | | | | |
|---|---|---|---|---|---|---|
| | | Ecoflac Plus A | Falcon B | PAB C | Ecobag D | Technoflex E |
| Initial | Initial | 99.7 | 98.7 | 99.3 | 97.5 | 98.5 |
| 6-month | 25°C | 100.7 | 99.5 | 100.2 | 97.4 | 98.4 |
| 12-month | 25°C | 98.4 | 98.7 | 95.5 | 101.1 | 99.6 |
| 18-month | 25°C | 100.1 | 100.9 | N/A | N/A | N/A |
| 24-month | 25°C | 98.7 | 99.5 | N/A | N/A | N/A |
| 6-month | 40°C | 99.2 | 100.4 | 99.5 | 102.2 | 98.0 |

Example 5: Comparison of leachable profile

As several peaks of leachables were observed during the studies, most intense leachable peaks observed on the UV and MS chromatograms were identified and semi quantified by comparison with a commercial calibrant. The identified compounds are consistent with the observed leachable compounds during screening leachable studies of associated containers and extractable study of card box.

The Compound Analytical Threshold (CAT) (i.e. minimum sensitivity of the method) required to reach a safety margin of 1.0 (safe level) is calculated for each targeted compound as follows: ratio of the Safety Index RfD divided by the Maximum Daily Dose with safety margin of 1. Those thresholds were calculated for each identified leachable compounds.

$$\text{CAT } (\mu g/mL) = \frac{RfD \text{ or } TTC \ (mg/kg/day)}{Max \ daily \ dose \ (mL/kg/day)} \times 1000 \ \mu g/mg$$

Identified leachable compounds are compared with the percentage of CAT obtained instead of their amount. Table 9 summarizes results per packaging over 24 month of storage and highlights only 4 identified leachable compounds in Ecoflac Plus containers while 5 and 6 in the others. Regardless to the packaging system, all concentrations of identified leachable compounds were found below their CAT values considering the current approved toxicological thresholds. T6 month time point at 40°C represents consequently the current worst case leachable profile versus T24 month stability point at 25°C.

Table 9: Comparison of already identified leachable profile of Ecoflac Plus with other containers

| Packaging | | Time point | Storage temperature | % CAT for already identified leachable | | | | | |
| --- | --- | --- | --- | --- | --- | --- | --- | --- | --- |
| | | | | Benzaldehyde | Vanillin | Caprolactam | Benzyl alcohol | Di-N-butylamine | Metilox Acid |
| A | **EcoFlac** Plus | 12-month | at 25°C | 0.00 | 0.04 | N.A. | 0.09 | 2.27 | N.A. |
| | | 24-month | at 25°C | 0.01 | 0.07 | N.A. | 0.13 | 1.28 | N.A. |
| | | 6-month | at 40°C | 0.01 | 0.60 | N.A. | 0.45 | 3.93 | N.A. |
| B | **FALCON** | 12-month | at 25°C | 0.00 | 0.00 | N.A. | 0.02 | 0.00 | 2.45 |
| | | 24-month | at 25°C | 0.00 | 0.00 | N.A. | 0.06 | 0.00 | 1.47 |
| | | 6-month | at 40°C | 0.03 | 0.30 | N.A. | 0.09 | 0.00 | 1.26 |
| D | **ECOBAG** | 12-month | at 25°C | 0.00 | 0.00 | 3.48 | 0.04 | 0.48 | 1.77 |
| | | 24-month | at 25°C | N.A. | N.A. | N.A. | N.A. | N.A. | N.A. |
| | | 6-month | at 40°C | 0.02 | 0.32 | 4.23 | 0.38 | 0.87 | 0.04 |
| E | **TECHNOFLEX** | 12-month | at 25°C | 0.04 | 0.00 | 1.72 | 0.00 | 0.14 | 12.83 |
| | | 24-month | at 25°C | N.A. | N.A. | N.A. | N.A. | N.A. | N.A. |
| | | 6-month | at 40°C | 0.06 | 0.00 | 4.24 | 0.02 | 0.18 | 9.95 |

For unknown leachable component, the Analytical Evaluation Threshold (AET) is considered. The AET is the threshold at or above which leachable should be characterized and reported for toxicological assessment (USP <1664>). The Estimated AET is calculated using the following formula:

$$\text{Estimated AET (µg/mL)} = \frac{Safety\ concern\ threshold\ (\mu g/day)^{(1)}}{Maximum\ daily\ dose\ volume\ (\text{mL/day})^{(2)}} = \frac{50\ \mu g\ /day}{588\ mL\ /day} = 0.085\ \text{µg/mL}$$

(1): Suggested general toxicity limit according to PQRI
(2): Maximum daily dose volume (mL /day) for 70 kg patient

For general toxicity: AET (µg/mL) = estimated AET x 50% = 0.042 µg/mL (42 µg/L)

The Safety Concern Threshold (SCT) value suggested at 50 µg/day by PQRI is not yet accepted by FDA and could be challenged by authorities. The conservative approach would be to use a 5 µg/day limit which would lead to divide by ten the AET value.

Regarding unidentified compounds, their concentrations were semi quantified using dexmedetomidine as external calibrant and compared to an AET calculated using a 50 µg/day SCT. With this toxicological threshold, no concentration of unidentified peak were found higher than this AET. As packaging discrimination parameters, Table 10 details the number of unknown peak observed above the conservative AET (with a SCT at 5 µg/day) and the global amount of all the unknowns. It highlights again the best results for Ecoflac container at 25°C over 24-month.

Table 10: Comparison of unknown leachable results of Ecoflac Plus with other containers

| Packaging | | Time point | Storage condition | Number of unknowns (> SCT at 5 µg/day) | ∑Amount (µg/L) |
|---|---|---|---|---|---|
| **A** | **ECOFLAC PLUS** | 12-month | at 25°C | 1 | 5 |
| | | 24-month | at 25°C | 0 | 0 |
| | | 6-month | at 40°C | 8 | 86 |
| **B** | **FALCON** | 12-month | at 25°C | 3 | 19 |
| | | 24-month | at 25°C | 1 | 8 |
| | | 6-month | at 40°C | 6 | 62 |
| **C** | **PAB** | 12-month | at 25°C | 10 | 291 |
| | | 24-month | at 25°C | N.A. | N.A. |
| | | 6-month | at 40°C | N.A. | N.A. |
| **D** | **ECOBAG** | 12-month | at 25°C | 5 | 109 |
| | | 24-month | at 25°C | N.A. | N.A. |
| | | 6-month | at 40°C | 12 | 321 |
| **E** | **TECHNOFLEX** | 12-month | at 25°C | 7 | 117 |
| | | 24-month | at 25°C | N.A. | N.A. |
| | | 6-month | at 40°C | 10 | 150 |

**Claims**

1. Container comprising a compartment filled with an aqueous solution of dexmedetomidine, wherein at least the inner surface of said compartment which is in contact with the aqueous solution of dexmedetomidine when the container is in upright position, is made of low density polyethylene (LDPE).

2. Container according to claim 1, wherein said LDPE has a density in the range of from 0.910-0.937 g/cm$^3$.

3. Container according to any of the preceding claims, wherein the compartment is sealingly closed.

4. Container according to claim 3, wherein

   (i) the entire inner layer of the sealed compartment of the container is made of LDPE, or
   (ii) the sealed compartment of the container consists of a single LDPE layer, or
   (iii) a separate sealing member is attached for closure of the compartment of the container, wherein preferably the separate sealing member has an inner layer made of LDPE or the sealing member consists of LDPE.

5. Container according to any of the preceding claims, wherein the LDPE is free of stabilizers and plasticizers, preferably the LDPE is free of any additives.

6. Container according to any of the preceding claims, which is made by applying an injection-molding technique, or blow-molding technique, preferably the container is made by applying the blow-fill-seal technique.

7. Container according to any of the preceding claims, wherein the compartment has a volume in the range of between at least 10 ml and 500 ml, and/or wherein the compartment has a wall thickness of at least 200 $\mu$m, preferably of at least 250 $\mu$m.

8. Container according to any of the preceding items, which is provided with a cap.

9. Container according to any of the preceding claims, wherein the aqueous dexmedetomidine solution is an aqueous dexmedetomidine/NaCl-solution, preferably is a premixed, ready-to-use dexmedetomidine solution,.

10. Container according to any of the preceding claims, wherein the aqueous dexmedetomidine-solution has a concentration of dexmedetomidine in the range of from 2 $\mu$g/ml to 12 $\mu$g/ml.

11. Use of a container comprising a compartment with an inner wall surface made of low density polyethylene (LDPE), or comprising a compartment made of low density polyethylene (LDPE), respectively for storing an aqueous dexmedetomidine-solution, preferably an aqueous dexmedetomidine-solution having a concentration of dexmedetomidine in the range of from 4 $\mu$g/ml to 8 $\mu$g/ml.

12. Method for manufacturing a container comprising a sealed compartment filled with an aqueous solution of dexmedetomidine, wherein the container is optionally provided with a cap, comprising the following steps:

   (i) Providing a container comprising a compartment with an inner surface made of low density polyethylene (LDPE);
   (ii) Filling the compartment of the container with the aqueous solution of dexmedetomidine;
   (iii) Sealing the filled compartment of the container.
   (iv) Optionally adding a cap on the top of the container

13. Method according to claim 12, further comprising an additional step (v) of sterilizing the filled, sealed and optionally capped container, optionally followed by a step (vi) of labelling the container.

14. Method according to claim 12 or 13, wherein the container is as defined in any of claims 1 to 8, and/or the aqueous solution of dexmedetomidine is as defined in any of claims 9 to 10.

15. Premixed aqueous solution of dexmedetomidine, filled in a container comprising a compartment such that an inner surface of the compartment of the container which may come into contact with the aqueous solution of dexmedetomidine is made of low density polyethylene (LDPE), for use in a medical treatment administrated parentally

## Fig. 1

EP 4 085 891 A1

## Fig. 2

Europäisches
Patentamt

European
Patent Office

Office européen
des brevets

# EUROPEAN SEARCH REPORT

Application Number

EP 21 17 2306

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/184188 A1 (SLYPHARMA LLC [US]) 26 October 2017 (2017-10-26) * page 11, paragraph 3 - page 12, paragraph 3 * | 1-15 | INV. A61J1/00 A61K31/4174 |
| X | US 2019/183729 A1 (SURA SIVA PRASAD REDDY [IN] ET AL) 20 June 2019 (2019-06-20) * paragraph [0099] - paragraph [0109] * | 1,3-5, 11-15 | |
| X | US 2014/323517 A1 (WHELAN JOHN [US]) 30 October 2014 (2014-10-30) * paragraphs [0136], [0156] * | 1,3-5, 11-15 | |
| A | US 2008/308444 A1 (MCCLAIN KIMBERLY A [US] ET AL) 18 December 2008 (2008-12-18) * abstract; figures * | 1-15 | |
| A | EP 0 601 631 A1 (DSM NV [NL]) 15 June 1994 (1994-06-15) * page 2, line 13 - line 17 * | 1,2 | |

TECHNICAL FIELDS
SEARCHED (IPC)

A61J
A61K

The present search report has been drawn up for all claims

| Place of search | Date of completion of the search | Examiner |
|---|---|---|
| The Hague | 14 October 2021 | Kousouretas, Ioannis |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 17 2306

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2021

| Patent document cited in search report | | Publication date | Patent family member(s) | | Publication date |
|---|---|---|---|---|---|
| WO 2017184188 | A1 | 26-10-2017 | AR | 108806 A1 | 26-09-2018 |
| | | | AU | 2016403510 A1 | 15-11-2018 |
| | | | EP | 3429574 A1 | 23-01-2019 |
| | | | US | 9649296 B1 | 16-05-2017 |
| | | | US | 9717796 B1 | 01-08-2017 |
| | | | WO | 2017184188 A1 | 26-10-2017 |
| | | | ZA | 201807466 B | 26-02-2020 |
| US 2019183729 | A1 | 20-06-2019 | NONE | | |
| US 2014323517 | A1 | 30-10-2014 | US | 2014323517 A1 | 30-10-2014 |
| | | | US | 2018193464 A1 | 12-07-2018 |
| US 2008308444 | A1 | 18-12-2008 | AT | 473175 T | 15-07-2010 |
| | | | EP | 2003064 A1 | 17-12-2008 |
| | | | ES | 2348628 T3 | 09-12-2010 |
| | | | JP | 2008307361 A | 25-12-2008 |
| | | | PL | 2003064 T3 | 31-12-2010 |
| | | | PT | 2003064 E | 07-10-2010 |
| | | | US | 2008308444 A1 | 18-12-2008 |
| | | | US | 2010326868 A1 | 30-12-2010 |
| | | | US | 2013334165 A1 | 19-12-2013 |
| | | | US | 2015144586 A1 | 28-05-2015 |
| | | | US | 2020163832 A1 | 28-05-2020 |
| | | | US | 2020163833 A1 | 28-05-2020 |
| | | | WO | 2008153581 A1 | 18-12-2008 |
| EP 0601631 | A1 | 15-06-1994 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10632043 B
- WO 2019164765 A
- US 9320712 B2
- US 9649296 B1
- US 9717796 B1
- WO 2017184188 A1
- JP 5921928 B
- US 2020138788 A1